# EUROPEAN PATENT APPLICATION

(11) **EP 3 510 996 A1**
(43) Date of publication of application: **17.07.2019**
(21) Application number: 18151209.6
(22) Date of filing: 11.01.2018
(51) Int. Cl.: A61K 9/16, A61K 9/20, A61K 31/44, A61P 7/02

(54) **PHARMACEUTICAL COMPOSITIONS OF BETRIXABAN**

(71) Applicant: Sandoz AG, 4056 Basel (CH)
(72) Inventor: FELZMANN, Wolfgang, 6250 Kundl (AT); RANEBURGER, Johannes, 6250 Kundl (AT)
(74) Representative: Kluschanzoff, Harald

(57) **Abstract**

The present invention relates to pharmaceutical compositions comprising betrixaban in a compacted form, processes for their preparation and the use of said pharmaceutical compositions in the treatment of disease conditions characterized by undesired thrombosis.

## Description

### FIELD OF INVENTION

The present invention relates to pharmaceutical compositions comprising betrixaban in a compacted form, processes for their preparation and the use of said pharmaceutical compositions in the treatment of disease conditions characterized by undesired thrombosis.

### BACKGROUND OF THE INVENTION

Betrixaban, N-(5-chloropyridin-2-yl)-2-([4-(N,N-dimethylcarbamimidoyl)benzoyl]amino)-5-methoxybenzamide, is a Factor Xa inhibitor for the prevention and treatment of venous thrombosis. It can be represented by the following chemical structure:

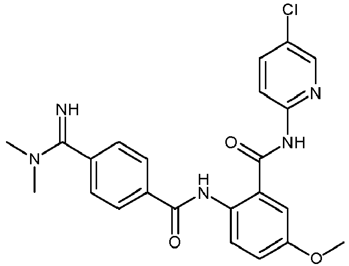

Betrixaban maleate is a well-soluble API belonging to BCS class III. The preferred mode of administration is via capsules. However, it was found that mixtures of betrixaban maleate and excipients filled in capsules show unacceptably slow dissolution behavior both for crystalline and amorphous API.

WO 2017/208169 and WO 2017/211779 both disclose solid dispersions comprising amorphous betrixaban maleate. Amorphous solid dispersions are generally known to improve solubility of drug products as the substrate is dispersed in a thin layer on the inert surface of a dispersing agent. Due to the amorphous state, no crystal energy needs to be overcome in the dissolution process resulting in faster dissolution kinetics and higher solubility. However, in solid dispersions the dispersing agent needs to be pharmaceutically acceptable. Furthermore, dispersant and dispersing agent need to be brought into close contact in a separate, often costly process step like spray drying, ball milling etc. known to those skilled in the art. A separate release of this material is required in most cases, adding further costs. Furthermore solid dispersions often inherit non-preferred properties of the dispersing agent like low bulk density, low flowability which complicates further processing.

Therefore the object of the present invention is the provision of a pharmaceutical composition of betrixaban allowing fast dissolution and high bioavailability, while at the same time avoiding the disadvantages of the above described solid dispersion.

### SUMMARY OF THE INVENTION

The present invention relates to pharmaceutical compositions comprising betrixaban in a compacted form, processes for their preparation and the use of said pharmaceutical compositions in the treatment of disease conditions characterized by undesired thrombosis.

Surprisingly, it was found that subjecting the bulk mixture of API and excipients to a compaction process, which is cheap pharmaceutical processing step, especially in comparison to the costly preparation of a solid dispersion, leads to an improved dissolution behavior.

### Definitions

As used herein, the term "compaction" refers to any process where the bulk density of a solid or a mixture of solids is increased after applying the process.

As used herein, the term "compacted form" refers to the product of a compaction process having the bulk density of a solid or a mixture of solids increased after applying the process.

As used herein, the term "dry compaction" refers to any process having the bulk density of a solid or a mixture of solids increased after applying the process which includes the application of pressure.

As used herein, the term "dry-granulation" used herein refers to a preparation wherein the dry formulation containing betrixaban or a salt thereof and further ingredients for a pharmaceutical dosage form have been processed by compacting into compacts, preferably using roller compactor or tableting machine by using slugging tooling or regular tableting tooling and subsequently milling - crushing and sizing these compacts into dry granulate.

As used herein, the term "wet-granulation" refers to a preparation wherein a bulk mixture containing betrixaban or a salt thereof and further ingredients for a pharmaceutical dosage are wetted using a pharmaceutically acceptable solvent, optionally in the presence of a suitable binder in an agitator leading to aggregation of the primary particles to form granules, followed by a subsequent drying step.

### BRIEF DESCRIPTION OF DRAWINGS

**Figure 1** illustrates the dissolution profiles of the uncompacted betrixaban maleate, the dry-compacted amorphous and crystalline betrixaban maleate and the amorphous solid dispersion of betrixaban maleate.
**Figure 2** illustrates the dissolution profiles of dry-compacted betrixaban salts: maleate, citrate and hydrobromide.

### DETAILED DESCRIPTION OF THE INVENTION

In a first embodiment the present invention relates to a pharmaceutical composition comprising betrixaban in a compacted form.

Regarding the compacted form it can be obtained by using wet or dry processing methods for the compaction process.

In certain embodiments the pharmaceutical composition is prepared by wet processing methods, such as, but not limited to, wet granulation methods.

In a more preferred embodiment the pharmaceutical composition is prepared by dry compaction, such as, but not limited to, direct compression or dry granulation methods. An example of dry granulation is roller compaction.

The dry compaction process involves applying pressure, preferably 10 -1000 bar, more preferably 50 - 800 bar, even more preferably 100 - 500 bar, most preferable 200 - 400 bar.

The above mentioned processes can be carried out using crystalline or amorphous form of a pharmaceutically acceptable salt of betrixaban, such as but not limited to, hydrochloride, hydrobromide, citrate, maleate, fumarate, benzoate, acetate, propionate, formiate, hydrogen sulfate, sulfate, lactate, preferably maleate, hydrochloride, hydrobromide, citrate, most preferably maleate.

The pharmaceutical composition of the invention may comprise one or more pharmaceutical excipients.

The one or more pharmaceutically acceptable excipient(s) is/are preferably selected from the group consisting of carriers, fillers, diluents, lubricants, glidants, sweeteners, stabilizing agents, solubilizing agents, antioxidants and preservatives, flavouring agents, binders, colorants, osmotic agents, buffers, surfactants, disintegrants, granulating agents, coating materials and combinations thereof. In a preferred embodiment, one or more pharmaceutically acceptable excipient(s) is/are selected from the group consisting of diluents, binders, disintegrants, lubricants and glidants.

Preferred fillers are selected from lactose, dextrose, cellulose, preferably dextrose.

Preferred desintegrants and lubricants are cross-carmellose and Mg-stearate.

In a preferred embodiment the pharmaceutical composition comprises dextrose, cross-carmellose and Mg-stearate as excipient.

The pharmaceutical composition of the present invention is preferably an oral solid dosage form, such as a tablet or a capsule.

In cases where the dosage form is a tablet, it can be coated or uncoated, single- or multilayered.

In the cases where the oral dosage form is a capsule, an optional homogenization step such as crushing, grinding, sieving, separating, classifying known to those skilled in the art can be used to provide material of a suitable particle distribution. The homogenized mixture can then be filled into suitable capsules, such as, but not limited to hard-gelatin capsules, HPMC capsules.

Preferred dosage form of the present invention is a hard gelatine capsule.

Preferably, the pharmaceutical composition comprises an amount of 40, 60 or 80 mg of the crystalline betrixaban salts of the invention, calculated as betrixaban free base.

### Preparation process

The present invention also relates to a process for the preparation of a pharmaceutical composition comprising betrixaban in a compacted form.

In a preferred embodiment the process is dry compaction.

The dry compaction process involves applying pressure, preferably 10 -1000 bar, more preferably 50 - 800 bar, even more preferably 100 - 500 bar, most preferable 200 - 400 bar.

### Use

The present invention also relates to the use of said pharmaceutical composition in the treatment of disease conditions characterized by undesired thrombosis.

### EXAMPLES

Dissolution data was obtained using an Agilent 708-DS water bath and 0.01M HCl as the dissolution medium at 37°C. Concentration measurement was done using an Agilent Cary 8454 photometer, with samples being taken in 2 minute intervals and measured at 275 nm wavelength.

### Comparative Example 1: Preparation of a capsule from form I of betrixaban maleate without compaction

440.29 mg crystalline betrixaban maleate (form I), 1683.55 mg dextrose monohydrate, 66.34 mg crosscarmellose sodium and 11.13 mg magnesium stearate were weighed together into a 100 mL glass vessel and homogenized for 5 minutes in a turbular mixer.

This bulk mixture was then filled into size 1 hard-gelatine capsules a 200.1 mg each.

### Comparative Example 2: Preparation of a capsule from amorphous betrixaban maleate solid dispersion without compaction

480.21 mg amorphous betrixaban maleate solid dispersion on HPMC (1:1), as described in WO2017/211779, 918.31 mg dextrose monohydrate, 36.6 mg crosscarmellose sodium and 6.04 mg magnesium stearate were weighed together into a 100 mL glass vessel and homogenized for 5 minutes in a turbular mixer.

This bulk mixture was then filled into size 1 hard-gelatine capsules a 240.1 mg each.

### Comparative Example 3: Preparation of a capsule from amorphous betrixaban maleate without compaction

440.21 mg spray-dried betrixaban maleate (amorphous), 1683.09 mg dextrose monohydrate, 66.10 mg crosscarmellose sodium and 11.03 mg magnesium stearate were weighed together into a 100 mL glass vessel and homogenized for 5 minutes in a turbular mixer.

This bulk mixture was then filled into size 1 hard-gelatine capsules a 200.1 mg each.

### Example 1: Preparation of a capsule from dry granulated form I of betrixaban maleate

251.89 mg crystalline betrixaban maleate (form I), 375.34 mg dextrose monohydrate, 37.62 mg crosscarmellose sodium and 5.33 mg magnesium stearate were weighed together into a 100 mL glass vessel and homogenized for 5 minutes in a turbular mixer.

The bulk mixture was then checked for homogeneity and then compacted to 3 mm slugs using a Röltgen Flexitab S applying 200 bar pressure. The produced slugs were then disintegrated in a mortar. This granulated bulk mixture was then filled into size 1 hard-gelatine capsules a 133.7 mg each.

### Example 2: Preparation of a capsule from dry granulated amorphous betrixaban maleate

440.21 mg amorphous betrixaban maleate, 1683.09 mg dextrose monohydrate, 66.10 mg crosscarmellose sodium and 11.03 mg magnesium stearate were weighed together into a 100 mL glass vessel and homogenized for 5 minutes in a turbular mixer.

The bulk mixture was then checked for homogeneity and then compacted to slugs using a Röltgen Flexitab S applying 280 bar pressure. The produced slugs were then disintegrated in a mortar. This granulated bulk mixture was then filled into size 1 hard-gelatine capsules a 200.1 mg each.

### Example 3: Preparation of a tablet from amorphous betrixaban maleate

440.21 mg amorphous betrixaban maleate, 1683.09 mg dextrose monohydrate, 66.10 mg crosscarmellose sodium and 11.03 mg magnesium stearate were weighed together into a 100 mL glass vessel and homogenized for 5 minutes in a turbular mixer.

The bulk mixture was then checked for homogeneity and then pressed to tablets in portions of 200.1 mg each using a Röltgen Flexitab S applying 280 bar pressure.

### Example 4: Preparation of a capsule from dry granulated betrixaban citrate

5.0 g betrixaban free base was dissolved in 100 mL acetone. Then 2.44 g citric acid monohydrate were added. After stirring for 20 h, the suspension was filtered and the residue washed with 10 mL acetone. The product was dried in vacuo at room temperature to give 6.12 g betrixaban citrate.

285.58 mg betrixaban citrate, 375.04 mg dextrose monohydrate, 37.50 mg crosscarmellose sodium and 5.3 mg magnesium stearate were weighed together into a 100 mL glass vessel and homogenized for 5 minutes in a turbular mixer.

The bulk mixture was then checked for homogeneity and then compacted to slugs using a Röltgen Flexitab S applying 200 bar pressure. The produced slugs were then disintegrated in a mortar. This granulated bulk mixture was then filled into size 1 hard-gelatine capsules a 140.7 mg each.

### Example 5: Preparation of a capsule from dry granulated betrixaban hydrobromide

5.0 g betrixaban free base was dissolved in 100 mL ethanol. Then 2.48 g 48% hydrobromic acid were added. After stirring for 20 h, the suspension was filtered and the residue washed with 10mL ethanol. The product was dried in vacuo at room temperature to give 4.48 g betrixaban hydrobromide.
236.31 mg betrixaban hydrobromide, 375.14 mg dextrose monohydrate, 37.44 mg crosscarmellose sodium and 5.32 mg magnesium stearate were weighed together into a 100 mL glass vessel and homogenized for 5 minutes in a turbular mixer.

The bulk mixture was then checked for homogeneity and then compacted to slugs using a Röltgen Flexitab S applying 200 bar pressure. The produced slugs were then disintegrated in a mortar. This granulated bulk mixture was then filled into size 1 hard-gelatine capsules a 130.8 mg each.

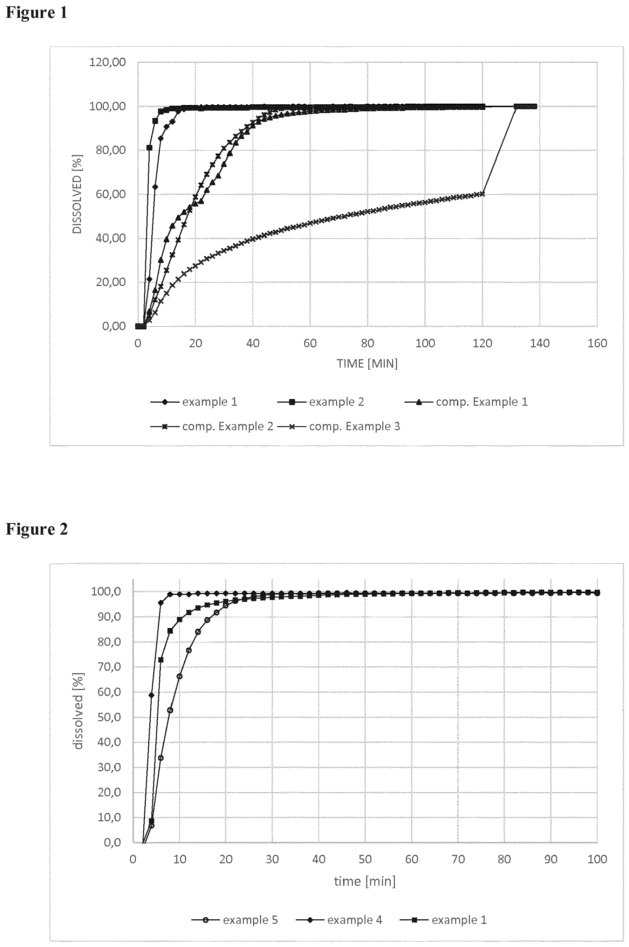

Figure 1 shows that both amorphous and crystalline API without compaction (Comparative Examples 1 and 3) show a surprisingly sluggish dissolution behavior. Using a solid dispersion (Comparative Example 2) leads to a more stable dissolution profile. However, when compacted material is used, both crystalline (Example 1) and amorphous (Example 2) API give a highly accelerated dissolution profile.

Figure 2 shows that this improved dissolution behavior seen upon compaction is not only true for betrixaban maleate, but also for betrixaban citrate (Example 4) and betrixaban hydrobromide (Example 5).

## Claims

1. A pharmaceutical composition comprising betrixaban in a compacted form.

2. A pharmaceutical composition according to claim 1 wherein betrixaban is in the form of a pharmaceutically suitable salt.

3. A pharmaceutical composition according to claim 2 wherein the salt is betrixaban maleate.

4. A pharmaceutical composition according to claim 3 wherein betrixaban maleate is in an amorphous form.

5. A pharmaceutical composition according to any one of the preceding claims wherein the compacted form is obtained by dry-compaction.

6. A pharmaceutical composition according to any one of the preceding claims wherein the compacted form is obtained by dry-granulation.

7. A pharmaceutical composition comprising betrixaban maleate in an amorphous form wherein the compacted form is obtained by dry-granulation.

8. Dosage form comprising the pharmaceutical composition according to any one of the preceding claims wherein the dosage form is a capsule.

9. Process for the preparation of the pharmaceutical composition of any one of the preceding claims comprising the steps of:
a) blending betrixaban and pharmaceutically acceptable excipients to give a bulk mixture,
b) compacting the bulk mixture.

10. Process according to any one of claims 9wherein pressure is applied during compaction.

11. Process according to claim 10 wherein the applied pressure is between 10 and 1000 bar, preferably between 50 and 800 bar, more preferably between 100 and 500 bar, most preferably between 200 and 400 bar.

12. Process according any of claims9-11 wherein the mixture obtained from step b) is homogenized and filled into a capsule.

13. Product obtained by any one of claims 9-12.

14. Use of the pharmaceutical composition of claims 1-7 in the treatment of disease conditions **characterized by** undesired thrombosis.
